# EUROPEAN PATENT APPLICATION

(11) **EP 4 155 302 A1**
(43) Date of publication of application: **29.03.2023**
(21) Application number: 21828887.6
(22) Date of filing: 18.06.2021
(51) Int. Cl.: C07D 401/12, C07D 401/14, C07D 405/14, C07D 413/14, C07D 409/14, C07D 417/14

(54) **PREPARATION METHOD FOR FUSED PYRAZOLE-TYPE COMPOUND**

(30) Priority: 23.06.2020 CN 202010581690
(71) Applicant: Shanghai Meiyue Biotech Development Co., Ltd., Pudong New District, Shanghai 201206 (CN)
(72) Inventor: YE, Guozhong, Shanghai 201206 (CN); TIAN, Yong, Shanghai 201206 (CN); FU, Shikang, Shanghai 201206 (CN); SUN, Zongguo, Shanghai 201206 (CN); CHEN, Yongkai, Shanghai 201206 (CN); WANG, Chaodong, Shanghai 201206 (CN)
(74) Representative: Gille Hrabal Partnerschaftsgesellschaft mbB Patentanwälte
(86) International application number: PCT/CN2021/100951
(87) International publication number: WO 2021/259163

(57) **Abstract**

The present disclosure provides a one-pot method for preparing a fused pyrazole-type compound substituted at nitrogen on 2-position, specifically an indazole-type compound substituted at nitrogen on 2-position. In the method, after azidizing a halogen in a substrate, without post-processing, R-NH₂ can be directly added to proceed a ring-closing reaction with an aldehyde group, so as to give the fused pyrazole-type compound substituted at nitrogen on 2-position. For example, by using 2-fluoro-4 methoxy-5-nitro benzaldehyde as a raw material, the indazole-type compound substituted at nitrogen on 2-position can be obtained via azidation and ring-closing steps, and then a series of indazole derivatives can be produced by means of a hydrogenation reaction and a condensation reaction. The present method is convenient, easy to operate, and generally applicable to various substrate structures, thereby suitable for large-scale production.

## Description

The present application claims priority to Chinese Patent Application No. 202010581690.X filed before China National Intellectual Property Administration on Jun. 23, 2020 and entitled "PREPARATION METHOD FOR FUSED PYRAZOLE-TYPE COMPOUND", which is incorporated herein by reference in its entirety.

### TECHNICAL FIELD

The present disclosure relates to the field of organic synthesis, and particularly to a method for preparing a fused pyrazole-type compound.

### BACKGROUND

Fused pyrazole compounds with substitution at the nitrogen on 2-position, especially indazole compounds with substitution at the nitrogen on 2-position, are typically prepared by substitution of indazole-type substrates with alkyl halides at present. However, such methods have been proven significantly deficient in that: 1) such methods have poor selectivity, leading to a mixture product of positional isomers with substitution at the N on 1-position and 2-position in the pyrazole ring respectively and thus to low yields; 2) the positional isomers above are difficult to post-treat and separate due to their similar properties such as polarity, and column chromatography is often required for purification and separation, making it difficult for industrial production; and 3) such reactions, via an SN₂ mechanism, would hardly proceed if the alkyl produces high steric hindrance.

Such a method through substitution was also described in patent reference CN110835332A, in which column chromatography is required for achieving product 1c of the following scheme, with a yield of only 22%:

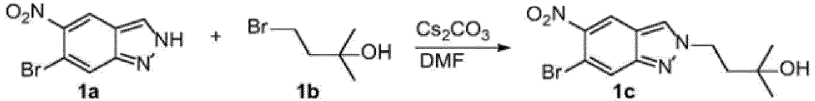

Also, patent reference CN109153665A reported another method for preparing such compounds:

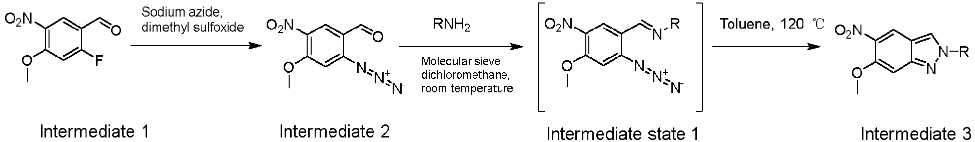

In the above method, the intermediate 2 and an amine (RNH₂) reacted with presence of dichloromethane/a molecular sieve to form the intermediate state 1, after removing the molecular sieve and dichloromethane, the intermediate state 1 was then heated in toluene solvent to give the intermediate 3. However, the above method is cumbersome in that dimethyl sulfoxide solution has a high boiling point, and that water and an organic solvent with a low boiling point should be added for extraction in the post-treatment. Also, this method requires concentration of the azide intermediate, involving safety risks such as explosion. Further, the method is complicated in that the molecular sieve and dichloromethane need to be removed before the production of intermediate 3 by heating in toluene in the above method, and column chromatography is still required for purification in the post-treatment, leading to low efficiency and unsuitability for large-scale production.

Therefore, there is a need to provide an improved preparation method so that a fused pyrazole-type compound can be prepared more conveniently, efficiently, economically and/or safely, thereby favoring large-scale production.

### SUMMARY

The present disclosure provides a method for preparing a compound of the following formula (I), a salt or another derivative thereof, comprising the following steps: 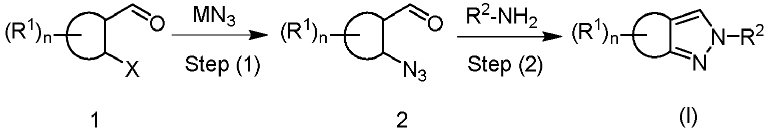
wherein, 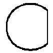 represents a ring system, such as a ring system selected from: C₃₋₄₀ cycloalkyl, 3- to 20-membered heterocyclyl, C₆₋₂₀ aryl and 5- to 20-membered heteroaryl;
each R¹ is the same or different and is independently selected from an organic group;
R² is selected from an organic group;
n is selected from an integer, such as 0, 1, 2, 3, 4, 5, 6, 7 and 8;
X is selected from a halogen, such as F, Cl, Br and I;
MN₃ is selected from an azide reagent, such as sodium azide, trimethylsilyl azide and diphenylphosphoryl azide;
the step (1) and/or step (2) are carried out in the presence of an alcohol-type solvent.

According to a preferred embodiment of the present disclosure, the step (2) may be carried out in the presence of an acid.

According to a preferred embodiment of the present disclosure, the step (2) may be carried out at a heating condition.

According to an embodiment of the present disclosure, the alcohol-type solvent may be selected from one, or a mixture of two or more of methanol, ethanol, *n*-propanol, isopropanol, *n*-butanol, isobutanol and *tert*-butanol, preferably from one, or a mixture of two or more of methanol, ethanol and isopropanol. Preferably, the alcohol-type solvent is an anhydrous solvent.

According to an embodiment of the present disclosure, the acid may be an organic acid or inorganic acid; for example, the acid is selected from one, or a mixture of two or more of formic acid, acetic acid, propionic acid, sulfuric acid (such as concentrated sulfuric acid) and *p*-toluenesulfonic acid.

According to an embodiment of the present disclosure, each R¹ is the same or different and is independently selected from a halogen, CN, OH, NO₂, and the following groups unsubstituted or optionally substituted with one, two or more R^{a}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, -O(CH₂)ₘR³, -S(CH₂)ₘR³, -NR⁴R⁵, -C(O)R⁶, -S(O)₂R⁶, -OC(O)R⁷, -OS(O)₂R⁷ and -S(O)(NR⁸)R⁹; wherein m is selected from an integer, such as 0, 1, 2, 3, 4, 5, 6, 7 and 8;
each R³ is the same or different, and is independently selected from C₁₋₄₀ alkyl, C₁₋₄₀ alkoxy, C₁₋₄₀ alkylthio, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
R⁴ and R⁵ are the same or different, and are each independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5-to 20-membered heteroaryl, 3- to 20-membered heterocyclyl, -C(O)R⁶ and -S(O)₂R⁶;
or R⁴ and R⁵, together with an N atom connected thereto, form 5- to 20-membered heteroaryl or 3- to 20-membered heterocyclyl;
each R⁶ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, -OR³, -SR³ and NR⁴R⁵;
each R⁷ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
each R⁸ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
each R⁹ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
R² is selected from the following groups unsubstituted or optionally substituted with one, two or more R^{b}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
each R^{a}, R^{b} is the same or different, and is independently selected from halogen, CN, OH, SH, oxo (=O or forming a nitrogen oxide), NO₂, and the following groups unsubstituted or optionally substituted with one, two or more R^{c}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, -OR³, -SR³, -NR⁴R⁵, -C(O)R⁶, -S(O)₂R⁶, -OC(O)R⁷, -OS(O)₂R⁷, -SOR⁷ and -S(O)(NR⁸)R⁹.

Each R^{c} is the same or different, and is independently selected from a halogen, CN, OH, SH, oxo (=O or forming a nitrogen oxide), NO₂, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl and C₁₋₄₀ haloalkyl.

According to an embodiment of the present disclosure, R¹ may be selected from NO₂, NH₂, and -OR³ unsubstituted or optionally substituted with one, two or more R^{a}.

According to an embodiment of the present disclosure, R³ may be selected from C₁₋₄₀ alkyl, such as C₁₋₆ alkyl.

According to an embodiment of the present disclosure, R² may be selected from the following groups, which are unsubstituted or optionally substituted with one, two or more R^{b}: -C₃₋₄₀ cycloalkyl-OH and -C₃₋₄₀ cycloalkyl-C₁₋₄₀ alkyl-OH, such as -C₄₋₆ cycloalkyl-OH and -C₄₋₆ cycloalkyl-C₁₋₆ alkyl-OH, such as 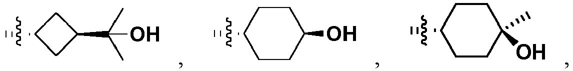 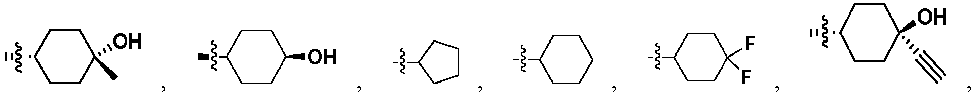 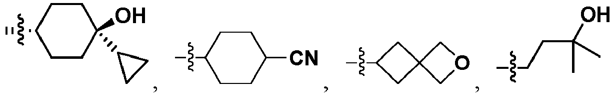 and

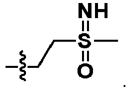

According to an embodiment of the present disclosure, n may be an integer selected from 0 to 4, preferably 2.

According to an embodiment of the present disclosure, where n is 2, two same or different R¹ may substitute at ortho or non-ortho positions.

According to the present disclosure, 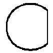 is substituted with the carbonyl group and X group at ortho atoms, preferably at ortho carbon atoms in the formula of compound 1.

According to the present disclosure, 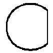 is substituted with the carbonyl group and azide group at ortho atoms, preferably at ortho carbon atoms in the formula of compound 2.

According to the present disclosure, 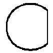 and the pyrazolyl group are fused in the structural formula of the compound of formula (I).

According to an embodiment of the present disclosure, after the reaction of the step (1) is completed, no post-treatment is required and the reaction of the step 2) can be directly carried out.

According to an embodiment of the present disclosure, in the step (2), the molar ratio of compound 2 to R-NH₂ is 1:(1-2), such as 1:(1-1.5), for example, 1:1, 1:1.1, 1:1.2, 1:1.3, 1:1.4 or 1:1.5.

According to an embodiment of the present disclosure, in the step (2), the molar ratio of compound 2 to the acid is 1:(1-5), such as 1:(1-4), for example, 1:1, 1:2, 1:3, 1:4 or 1:5.

According to an embodiment of the present disclosure, the reaction time of the step (2) may be 1-24 h, such as 2-18 h, for example, 2, 3, 4, 5, 6, 7, 8, 9, 10, 11, 12, 13, 14, 15, 16, 17 or 18 h.

According to an embodiment of the present disclosure, the reaction of the step (2) may be carried out at a temperature of 40-120 °C, such as 60-100 °C, for example, 70-85 °C.

According to a preferred embodiment of the present disclosure, after the reaction of the step (1) is completed, the reaction of the step (2) is directly carried out without isolating the compound of formula 2.

According to a preferred embodiment of the present disclosure, after the reaction of the step (2) is completed, the compound of formula (I) is isolated without using column chromatography.

According to a preferred embodiment of the present disclosure, after the reaction of the step (2) is completed, the compound of formula (I) can be isolated by using a method such as filtering the reaction system through celite and then concentrating the resulting filtrate, adding water to the reaction system and then filtering the resulting mixture, and adding water to the reaction system and then concentrating the resulting mixture.

According to an exemplary embodiment of the present disclosure, the method comprises the following steps: 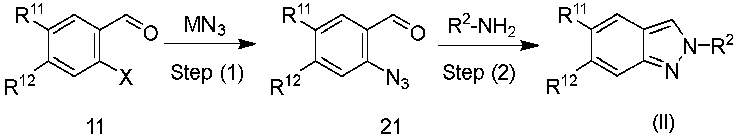
wherein, R¹¹ and R¹² are the same or different and are each independently selected from the definitions of R¹ as described above;
R², X and M are each independently as defined above.

According to a preferred embodiment of the present disclosure, R¹¹ is selected from NO₂.

According to a preferred embodiment of the present disclosure, R¹² is selected from methoxy.

According to a preferred embodiment of the present disclosure, R² is selected from

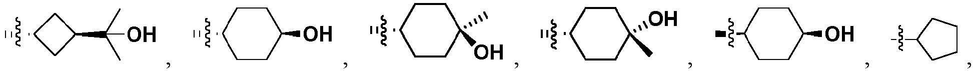

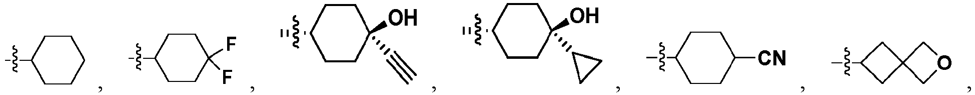

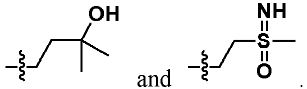

According to a preferred embodiment of the present disclosure, X is selected from F.

According to a preferred embodiment of the present disclosure, MN₃ is selected from NaN₃.

According to an embodiment of the present disclosure, the compound of formula (I) is preferably selected from the compound of formula (II).

According to a preferred embodiment of the present disclosure, the method is used for preparing an indazole-type compound, such as the compound of formula (II), a salt or another derivative thereof.

The present disclosure further provides use of the method in preparing an indazole derivative, such as an indazole derivative having inhibitory activity against IRAK.

The present disclosure further provides a method for preparing the derivative or salt of the compound of formula (I), comprising derivatizing a group in the compound of formula (I) or the salt thereof with a reagent after the above preparation method.

For example, the present disclosure provides a method for preparing a compound of formula 4 comprising:
(a1) subjecting a compound of formula (I-3) to a reduction reaction to give compound 3; and
(a2) subjecting compound 3 and Rₛ-COOH to a condensation reaction to give compound 4; 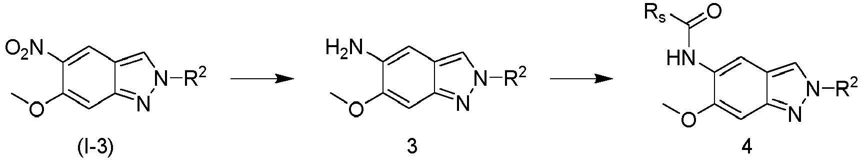 wherein, Rₛ is selected from the following groups unsubstituted or optionally substituted with one, two or more R^{a}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
R² and R^{a} are independently as defined above.

According to an exemplary embodiment of the present disclosure, Rₛ may be selected from

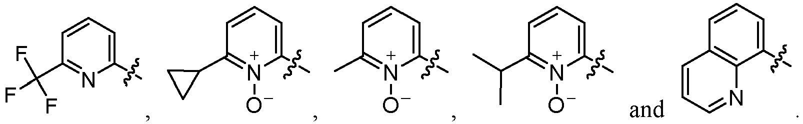

The present disclosure further provides a composition comprising a compound of formula 1, a compound of formula 2 and an alcohol-type solvent.

The present disclosure further provides a composition comprising a compound of formula 2, a compound of formula (I), an alcohol-type solvent and an acid.

The present disclosure further provides a composition comprising a compound of formula 1, a compound of formula 2, a compound of formula (I), an alcohol-type solvent and an acid.

Any one of the compositions described above may further optionally comprise or do not comprise at least one selected from the following components: MN₃ and R²-NH₂.

The compound of formula (I) of the present disclosure may be a racemate, a stereoisomer, a conformer or a tautomer.

An element in the structures of the compound of formula 1, the compound of formula 2 and the compound of formula (I) of the present disclosure may be optionally replaced by isotopes thereof; for example, ¹H may be replaced by ²H.

### Definitions and Description

Unless otherwise stated, the terms and description in the context of the present disclosure have the meanings set forth below.

The term "more" means 3 or more.

The term "halogen" refers to fluorine, chlorine, bromine or iodine.

The term "C₁₋₄₀ alkyl" preferably refers to a linear or branched saturated monovalent hydrocarbyl group having 1-40 carbon atoms. For example, "C₁₋₁₀ alkyl" refers to a linear or branched alkyl group having 1, 2, 3, 4, 5, 6, 7, 8, 9 or 10 carbon atoms and "C₁₋₆ alkyl" refers to a linear or branched alkyl group having 1, 2, 3, 4, 5 or 6 carbon atoms. The alkyl is, for example, methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or isomers thereof.

The term "C₂₋₄₀ alkenyl" preferably refers to a linear or branched monovalent hydrocarbyl comprising one or more double bonds and having 2-40 carbon atoms, preferably "C₂₋₁₀ alkenyl". The "C₂₋₁₀ alkenyl" preferably refers to a linear or branched monovalent hydrocarbyl comprising one or more double bonds and having 2, 3, 4, 5, 6, 7, 8, 9 or 10 carbon atoms, for example, having 2, 3, 4, 5 or 6 carbon atoms (i.e., C₂₋₆ alkenyl) or having 2 or 3 carbon atoms (i.e., C₂₋₃ alkenyl). It should be understood that in the case that the alkenyl comprises more than one double bond, the double bonds can be separated from one another or conjugated. The alkenyl is, for example, vinyl, allyl, (*E*)-2-methylvinyl, (*Z*)-2-methylvinyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl or 1-isopropylvinyl.

The term "C₂₋₄₀ alkynyl" refers to a linear or branched monovalent hydrocarbyl comprising one or more triple bonds and having 2-40 carbon atoms, preferably "C₂₋₁₀ alkynyl". The term "C₂₋₁₀ alkynyl" preferably refers to a linear or branched monovalent hydrocarbyl comprising one or more triple bonds and having 2, 3, 4, 5, 6, 7, 8, 9 or 10 carbon atoms, for example, having 2, 3, 4, 5 or 6 carbon atoms (i.e., "C₂₋₆ alkynyl") or having 2 or 3 carbon atoms ("C₂₋₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl or prop-2-ynyl.

The term "C₃₋₄₀ cycloalkyl" refers to a saturated monovalent monocyclic or bicyclic hydrocarbon ring or bridged cycloalkane having 3-40 carbon atoms, preferably "C₃₋₁₀ cycloalkyl". The term "C₃₋₁₀ cycloalkyl" refers to a saturated monovalent monocyclic or bicyclic hydrocarbon ring or bridged cycloalkane having 3, 4, 5, 6, 7, 8, 9 or 10 carbon atoms. The C₃₋₁₀ cycloalkyl may be a monocyclic hydrocarbyl such as cyclopropyl, cyclobutyl, cyclopentyl, cyclohexyl, cycloheptyl, cyclooctyl, cyclononyl or cyclodecyl, or may be a bicyclic hydrocarbyl such as a decahydronaphthalene ring. The cycloalkyl may be a spiro ring such as a spiro[3,3] ring, a spiro[3,4] ring, a spiro[3,5] ring, a spiro[4,4] ring, a spiro[4,5] ring, or a spiro[5,5] ring.

The term "3- to 20-membered heterocyclyl" refers to a saturated monovalent monocyclic or bicyclic hydrocarbon ring or bridged cycloalkane, which is a non-aromatic cyclic group with a total number of 3-20 (such as 3, 4, 5, 6, 7, 8, 9 and 10) ring atoms comprising 1-5 heteroatoms independently selected from N, O and S, preferably a "3- to 10-membered heterocyclyl". The term "3- to 10-membered heterocyclyl" refers to a saturated monovalent monocyclic or bicyclic hydrocarbon ring or bridged cycloalkane comprising 1-5, preferably 1-3, heteroatoms selected from N, O and S. The heterocyclyl may be connected to the rest of the molecule through any one of the carbon atoms or the nitrogen atom (if present). In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidinyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The heterocyclyl may be a spiro ring, for example, but not limited to, a spiro[3,3] ring such as a spiro[3,3] ring, a spiro[3,4] ring, a spiro[3,5] ring, a spiro[4,4] ring, a spiro[4,5] ring, or a spiro[5,5] ring, such as 2,7-diazaspiro[3,5]nonane, 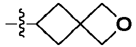 or 2,6-diazaspiro[3,4]octane. The ring containing nitrogen atoms may be partially unsaturated, i.e., it may contain one or more double bonds, for example, but not limited to, 2,5-dihydro-1*H*-pyrrolyl, 4*H*-[1,3,4]thiadiazinyl, 4,5-dihydrooxazolyl or 4*H*-[1,4]thiazinyl, or it may be benzo-fused, for example, but not limited to, dihydroisoquinolyl. According to the present disclosure, the heterocyclyl is non-aromatic. When the 3- to 20-membered heterocyclyl is connected to another group to form the compound of the present disclosure, the group may be connected to the carbon atom on the 3- to 20-membered heterocyclyl, or may be connected to the heteroatom on the 3- to 20-membered heterocyclyl. For example, when the 3- to 20-membered heterocyclyl is selected from piperazinyl, the group may be connected to the nitrogen atom on the piperazinyl. Alternatively, when the 3- to 20-membered heterocyclyl is selected from piperidinyl, the group may be connected to the nitrogen atom on the piperidinyl or the carbon atom in the para position.

The term "C₆₋₂₀ aryl" preferably refers to an aromatic or partially aromatic monovalent monocyclic, bicyclic or tricyclic hydrocarbon ring having 6-20 carbon atoms, preferably "C₆₋₁₄ aryl". The term "C₆₋₁₄ aryl" preferably refers to an aromatic or partially aromatic monovalent monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl or biphenyl, a ring having 9 carbon atoms ("C₉ aryl"), such as indanyl or indenyl, a ring having 10 carbon atoms ("C₁₀ aryl"), such as tetrahydronaphthyl, dihydronaphthyl or naphthyl, a ring having 13 carbon atoms ("C₁₃ aryl"), such as fluorenyl, or a ring having 14 carbon atoms ("C₁₄ aryl"), such as anthryl. When the C₆₋₂₀ aryl is substituted, it may be monosubstituted or polysubstituted. In addition, the substitution site is not limited, and may be, for example, ortho-, para- or meta-substitution.

The term "5- to 20-membered heteroaryl" refers to a monovalent aromatic monocyclic, bicyclic or tricyclic ring which has 5-20 ring atoms and comprises 1-5 heteroatoms independently selected from N, O and S, such as "5- to 14-membered heteroaryl". The term "5- to 14-membered heteroaryl" refers to a monovalent aromatic monocyclic, bicyclic or tricyclic ring which has 5, 6, 7, 8, 9, 10, 11, 12, 13 or 14 ring atoms, in particular 5, 6, 9 or 10 carbon atoms, comprises 1-5, preferably 1-3 heteroatoms independently selected from N, O and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like, and benzo derivatives thereof such as benzofuranyl, benzothienyl, benzoxazolyl, benzoisoxazolyl, benzimidazolyl, benzotriazolyl, indazolyl, indolyl, and isoindolyl; or pyridinyl, pyridazinyl, pyrimidinyl, pyrazinyl, triazinyl and the like, and benzo derivatives thereof such as quinolyl, quinazolinyl, and isoquinolyl; or azocinyl, indolizinyl, purinyl and the like, and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. When the 5- to 20-membered heteroaryl is connected to another group to form the compound of the present disclosure, the group may be connected to the carbon atom on the 5- to 20-membered heteroaryl ring, or may be connected to the heteroatom on the 5- to 20-membered heteroaryl ring. When the 5- to 20-membered heteroaryl is substituted, it may be monosubstituted or polysubstituted. In addition, the substitution site is not limited. For example, hydrogen connected to the carbon atom on the heteroaryl ring may be substituted, or hydrogen connected to the heteroatom on the heteroaryl ring may be substituted. When a heteroaryl group comprises an N atom, the 5- to 20-membered heteroaryl may also be oxidized to a nitrogen oxide thereof.

The term "oxo" refers to an oxide formed by oxidation of a carbon atom, nitrogen atom or sulfur atom in a substituent, such as a carbonyl group or a nitrogen oxide.

Unless otherwise stated, the heterocyclyl, heteroaryl or heteroarylene includes all possible isomeric forms thereof, such as positional isomers thereof. Thus, for some illustrative non-limiting examples, forms that involving substitutions at or bonding to other groups at one, two or more of positions 1, 2, 3, 4, 5, 6, 7, 8, 9, 10, 11, 12 and the like (if present) are included, including pyridin-2-yl, pyridinylene-2-yl, pyridin-3-yl, pyridinylene-3-yl, pyridin-4-yl and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl and pyrazol-5-yl.

### Beneficial Effects

The present disclosure provides a one-pot method for preparing a fused pyrazole-type compound with substitution at the nitrogen on 2-position, especially an indazole-type compound with substitution at the nitrogen on 2-position, which surprisingly avoids the safety risk in the separation of an azide intermediate.

Further, the overall yield of the compound of formula (I) in steps (1) and (2) of the present disclosure is above 80%, showing significantly elevated yield and cost efficiency.

In addition, as the present method does not necessarily involve complicated steps such as molecular sieve or column chromatography process in post-treatment for isolating product, it is more convenient and practicable, and suitable for large-scale and industrial production.

### DETAILED DESCRIPTION

The technical scheme of the present disclosure will be further illustrated in detail with reference to the following specific examples. It should be understood that the following examples are merely exemplary illustration and explanation of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared by known methods.

### Example 1: Preparation of trans-2-[3-(6-methoxy-5-nitro-indazol-2-yl)-cyclobutyl]-propyl-2-ol

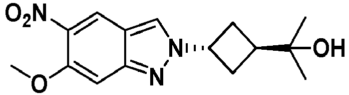

2-Fluoro-4-methoxy-5-nitrobenzaldehyde (10.00 g) and sodium azide (3.60 g) were added sequentially to a solution of methanol (100 mL). The reaction mixture was stirred at 20-30 °C for 16 h, without further treatment, to give a solution of 2-azido-4-methoxy-5-nitrobenzaldehyde in methanol (50.00 mmol, calculated according to 100% yield). To the above solution of 2-azido-4-methoxy-5-nitrobenzaldehyde in methanol were added 2-(3-amino-cyclobutyl)-*trans*-propyl-2-ol (4.66 g) and concentrated sulfuric acid (5.00 g, 98%). The system was incubated at reflux at a reaction temperature of 72-77 °C for 3 h. Water (40 mL) was added to the reaction system. The mixture was concentrated *in vacuo* until a certain volume (40 mL) was reached, and then cooled and filtered. The filter cake was dried to give *trans*-2-[3-(6-methoxy-5-nitro-indazol-2-yl)-cyclobutyl]-propyl-2-ol (10.80 g, 80.2% yield over two steps). ¹H NMR (400 MHz, CDCl₃): δ 8.65 (s, 1H), 8.37 (s, 1H), 7.31 (s, 1H), 5.08 (t, *J* = 7.17 Hz, 1H), 4.44 (s, 1H), 3.91 (s, 3H), 2.62-2.52 (m, 4H), 1.08 (d, *J* = 13.41 Hz, 7H); LCMS m/z = 306[M+H]⁺.

### Example 2: Preparation of trans-2-[3-(6-methoxy-5-amino-indazol-2-yl)-cyclobutyl]-propyl-2-ol

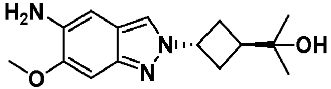

2-[3-(6-methoxy-5-nitro-indazol-2-yl)-cyclobutyl]-*trans*-propyl-2-ol (10.00 g) was added to anhydrous ethanol (100 mL), followed by palladium on carbon (0.50 g, anhydrous, 10% loading) in nitrogen atmosphere. The reaction system was purged with hydrogen, stirred in hydrogen atmosphere (at 1 standard atmosphere pressure) at 25-30 °C for 3 h, and filtered through celite. The filtrate was concentrated to give *trans*-2-[3-(6-methoxy-5-amino-indazol-2-yl)-cyclobutyl]-propyl-2-ol (8.73 g, 96.1% yield). ¹H NMR (400 MHz, DMSO): δ 7.90 (s, 1H), 6.87 (s, 1H), 6.62 (s, 1H), 4.87 ( p, *J* = 7.31 Hz, 1H), 4.58 (s, 2H), 4.37 (s, 1H), 3.82 (s, 3H), 2.48 (dd, *J* = 16.43, 8.83 Hz, 4H), 2.44-2.32 (m, 1H), 1.18-1.02 (m, 6H); LCMS m/z = 276[M+H]⁺.

### Example 3: Preparation of 6-trifluoromethyl-pyridine-2-carbomoylic acid {2-[3'-(1-hydroxy-1-methyl-ethyl)-cyclobutyl]-6-methoxy-2H-indazol-5-yl}-amide

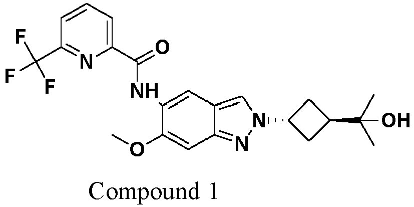

2-[3-(6-Methoxy-5-amino-indazol-2-yl)-cyclobutyl]-*trans*-propyl-2-ol (8.25 g) was added to tetrahydrofuran (60 mL), followed sequentially by 5-trifluoromethyl-2-pyridinecarboxylic acid (5.73 g), 2-(7-azabenzotriazole)-*N*,*N*,*N*',*N*'-tetramethyluronium hexafluorophosphate (12.55 g) and *N*,*N*-diisopropylethylamine (4.27 g). The reaction mixture was stirred at room temperature for 4 h. Water (120 mL) was added, and the resulting mixture was filtered. The filter cake was dried to give the compound 1 (11.49 g, 85.4% yield). ¹H NMR (400 MHz, CDCl₃): δ 10.71 (s, 1H), 8.81 (s, 1H), 8.50 (d, *J* = 8.0 Hz, 1H), 8.11 (t, *J* = 8.0 Hz, 1H), 7.89 (s, 1H), 7.86 (d, *J* = 6.8 Hz, 1H), 7.11 (s, 1H), 5.05-4.99 (m, 1H), 4.03 (s, 3H), 2.79-2.72 (m, 2H), 2.67-2.60 (m, 3H), 1.34 (s, 1H), 1.25 (s, 6H); LCMS m/z = 449.2[M+H]⁺.

### Example 4: Preparation of trans-4-(6-methoxy-5-nitro-indazol-2-yl)-cyclohexanol

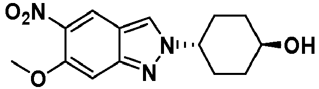

2-Fluoro-4-methoxy-5-nitrobenzaldehyde (10.00 g) and sodium azide (3.60 g) were added sequentially to a solution of isopropanol (100 mL). The reaction mixture was stirred at 20-30 °C for 16 h, without further treatment, to give a solution of 2-fluoro-4-methoxy-5-nitrobenzaldehyde in isopropanol (50.00 mmol, calculated according to 100% yield). To the solution of 2-azido-4-methoxy-5-nitrobenzaldehyde in isopropanol (i.e., Example 2, calculated according to 100% yield, i.e., 50.00 mmol) were added 4-amino-*trans*-cyclohexanol (4.66 g) and *p*-toluenesulfonic acid (8.60 g). The system was incubated at reflux at a reaction temperature of 76-82 °C for 16 h. Water (40 mL) was added to the reaction system. The mixture was concentrated *in vacuo* until a certain volume (40 mL) was reached, and then cooled and filtered. The filter cake was dried to give *trans*-4-(6-methoxy-5-nitro-indazol-2-yl)-cyclohexanol (12.29 g, 84.5% yield over two steps). ¹H NMR (400 MHz, DMSO): δ 8.58 (s, 1H), 8.38 (s, 1H), 7.25 (s, 1H), 4.74 (d, *J* = 3.60 Hz, 1H), 4.59-4.41 (m, 1H), 3.54 (dd, *J* = 10.34, 3.47 Hz, 1H), 3.91 (s, 3H), 2.09 (d, *J* = 9.93 Hz, 2H), 2.04-1.84 (m, 4H), 1.40 (q, 2H); LCMS m/z = 292[M+H]⁺.

### Example 5: Preparation of trans-4-(6-methoxy-5-amino-indazol-2-yl)-cyclohexanol

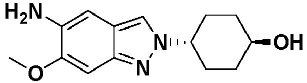

*Trans*-4-(6-methoxy-5-nitro-indazol-2-yl)-cyclohexanol (8.73 g) was added to anhydrous ethanol (100 mL), followed by palladium on carbon (0.50 g, anhydrous, 10% loading) in nitrogen atmosphere. The reaction mixture was purged with hydrogen, stirred in hydrogen atmosphere (at 1 standard atmosphere pressure) at 25-30 °C for 3 h, and filtered through celite. The filtrate was concentrated to give *trans*-4-(6-methoxy-5-amino-indazol-2-yl)-cyclohexanol (7.61 g, 97.2% yield). ¹H NMR (400 MHz, DMSO): δ 8.03 (s, 1H), 6.94 (s, 1H), 6.78 (s, 1H), 4.52 (s, 2H), 4.47 (s, 1H), 4.41-4.30 (m, 1H), 3.78 (s, 3H), 3.52 (m, 1H), 2.10 (m, 2H), 2.01-1.89 (m, 4H), 1.20 (q, 2H); LCMS m/z = 262[M+H]⁺.

### Example 6: Preparation of 6-cyclopropyl-1-oxy-pyridine-2-carbomoylic acid [2-(4'-hydroxy-trans-cyclohexyl)-6-methoxy-2H-indazol-5-yl]-amide

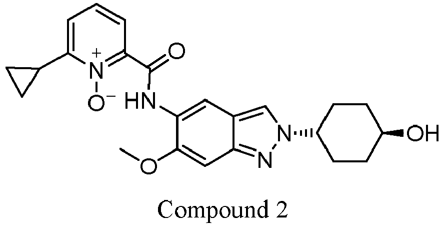

*Trans*-4-(6-Methoxy-5-amino-indazol-2-yl)-cyclohexanol (6.52 g) was added to tetrahydrofuran (50 mL), followed by sequentially adding 5-cyclopropyl-1-oxo-2-pyridinecarboxylic acid (4.93 g), 2-(7-azabenzotriazole)-*N*,*N*,*N*',*N*'-tetramethyluronium hexafluorophosphate (10.46 g) and *N*,*N*-diisopropylethylamine (3.55 g). The reaction system was stirred at room temperature for 4 h. Water (100 mL) was added, and the resulting mixture was filtered. The filter cake was dried to give the compound 2 (8.72 g, 82.6% yield). ¹H NMR (400 MHz, CDCl₃): δ 14.26 (s, 1H), 8.87 (s, 1H), 8.39 (d, *J* = 8.0 Hz, 1H), 7.82 (s, 1H), 7.39-7.35 (m, 1H), 7.04 (s, 2H), 4.37-4.29 (m, 1H), 4.04 (s, 3H), 3.83-3.75 (m, 1H), 2.88-2.80 (m, 1H), 2.28-2.25 (m, 2H), 2.18-2.15 (m, 2H), 2.09-2.00 (m, 2H), 1.58-1.48 (m, 2H), 1.29-1.26 (m, 2H), 0.84-0.82 (m, 2H); LCMS m/z = 423.2[M+H]⁺.

### Example 7: Preparation of cis-4-(6-methoxy-5-nitro-indazol-2-yl)-1-methyl-cyclohexanol

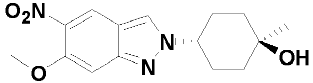

To a 20-L bilayer glass reactor were sequentially added anhydrous ethanol (7 L) and 2-fluoro-4-methoxy-5-nitrobenzaldehyde (995.00 g). The reactor was cooled to an internal temperature of 0-5 °C. Sodium azide (334.80 g) was added in batches. After the addition, the reaction mixture was stirred for 3 h, with the internal temperature of the reactor maintained at 0-5 °C. Then the temperature was gradually raised to 20-30 °C, and the reaction mixture was stirred at the temperature for 16 h without further treatment to give a solution of 2-azido-4-methoxy-5-nitrobenzaldehyde in ethanol (5.00 mol, calculated according to 100% yield).

The above solution of 2-azido-4-methoxy-5-nitrobenzaldehyde in ethanol (5.00 mol, calculated according to 100% yield) in the 20 L bilayer glass reactor was cooled, with the internal temperature of the reactor maintained at 0-5 °C. Acetic acid (900.00 g) and *cis*-4-amino-1-methylcyclohexanol (645.00 g) were added. The temperature was raised to 72-77 °C, and the reaction mixture was incubated for 5 h. The reaction system was cooled to 30-40 °C, and water (6 L) was then added. The reaction mixture was concentrated *in vacuo* until a certain volume (about 7 L) was reached. The reactor was cooled, with the internal temperature of the reactor maintained at 20-25 °C. The reaction mixture was filtered at reduced pressure. The filter cake was rinsed with a small amount of water, then added to a reactor, and recrystallized in ethanol and *n*-heptane. The crystal was collected by filtration and dried to give *cis*-4-(6-methoxy-5-nitro-indazol-2-yl)-1-methyl-cyclohexanol (1366.40 g, 89.6% yield). ¹H NMR (400 MHz, DMSO): δ 8.65 (d, J = 7.61 Hz, 1H), 8.38 (s, 1H), 7.27 (d, J = 10.99 Hz, 1H), 4.47 (d, J = 8.80 Hz, 1H), 4.35 (t, J = 5.05 Hz, 1H), 3.98-3.84 (m, 3H), 2.04 (tt, J = 13.79, 6.87 Hz, 4H), 1.73-1.51 (m, 4H), 1.23 (s, 3H); LCMS m/z = 306[M+H]⁺.

### Example 8: Preparation of cis-4-(6-methoxy-5-amino-indazol-2-yl)-1-methyl-cyclohexanol

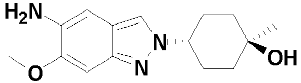

Anhydrous ethanol (13.5 L) and *cis*-4-(6-methoxy-5-nitro-indazol-2-yl)-1-methyl-cyclohexanol (1355.00 g) were sequentially added to a stainless steel hydrogenation pressure reactor (30 L). Nitrogen was constantly introduced into the reactor. A suspension of palladium on carbon in ethanol (40.00 g, anhydrous, 10% loading, prepared by mixing with 0.5 L of ethanol) was pumped into the reaction mixture. After the addition, the system was purged with nitrogen 3 times, followed by hydrogen 3 times. A certain hydrogen pressure (1 standard atmosphere pressure) was maintained in the system, and the internal temperature of the reactor was maintained at 20-25 °C. The reaction mixture was stirred for 3 h. The reaction system was filtered at a reduced pressure through a proper amount of celite. The filter cake was rinsed with a small amount of anhydrous ethanol. The filtrate was poured into a reactor, and concentrated *in vacuo* until a certain volume (about 3 L) was reached, with the internal temperature controlled at 30-40 °C. The temperature was then reduced to 0-5 °C, and *n*-heptane (20 L) was added. The mixture was stirred for 5 h and filtered. The filter cake was dried to give *cis*-4-(6-methoxy-5-amino-indazol-2-yl)-1-methyl-cyclohexanol (1160.64 g, 95.1% yield). ¹H NMR (400 MHz, DMSO): δ 7.88 (s, 1H), 6.84 (s, 1H), 6.61 (s, 1H), 4.56 (s, 2H), 4.42 (s, 1H), 4.38-4.21 (m, 1H), 3.81 (s, 3H), 1.71-1.47 (m, 4H), 2.03-1.93 (m, 4H), 1.21 (t, 3H); LCMS m/z = 276[M+H]⁺.

### Example 9: Preparation of quinoline-8-carbomoylic acid [2-(4'-hydroxy-4-methyl-cyclohexyl)-6-methoxy-2H-indazol-5-yl]-amide

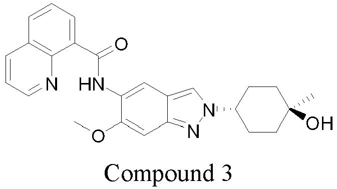

Anhydrous tetrahydrofuran (12 L) and *cis*-4-(6-methoxy-5-amino-indazol-2-yl)-1-methyl-cyclohexanol (1100.00 g) were sequentially added to a bilayer glass reactor (20 L). The internal temperature of the reactor was maintained at 20-25 °C. 8-Quinolinecarboxylic acid (688.64 g), 2-(7-azabenzotriazole)-*N*,*N*,*N*',*N*'-tetramethyluronium hexafluorophosphate (1551.38 g) and *N*,*N*-diisopropylethylamine (672.10 g) were sequentially added. After the addition, the reaction mixture was stirred for 3 h. Water (5 L) was added to the reaction system. The mixture was then warmed to 30-35 °C, concentrated *in vacuo* until a certain volume (7 L) was reached, cooled to 0-10 °C, stirred for another 5 h, and filtered. The filter cake was recrystallized in ethanol and methyl *tert*-butyl ether, and the crystal was collected by filtration and dried to give the compound 3 (1579.07 g, 91.7% yield). ¹H NMR (400 MHz, CDCl₃): δ 14.04 (s, 1H), 9.06-9.04 (m, 1H), 9.03 (s, 1H), 8.98-8.96 (m, 1H), 8.34-8.32 (m, 1H), 8.02-7.99 (m, 1H), 7.88 (s, 1H), 7.74 (t, *J* = 7.6 Hz, 1H), 7.57-7.54 (m, 1H), 7.11 (s, 1H), 4.42-4.41 (m, 1H), 4.11 (s, 3H), 2.28-2.15 (m, 4H), 1.90-1.87 (m, 2H), 1.76-1.65 (m, 2H), 1.45 (s, 1H), 1.41 (s, 3H); LCMS m/z = 431.1[M+H]⁺.

### Example 10: Preparation of 6-methyl-1-oxo-pyridine-2-carbomoylic acid [2-(4'-hydroxy-4-methyl-cyclohexyl)-6-methoxy-2H-indazol-5-yl]-amide

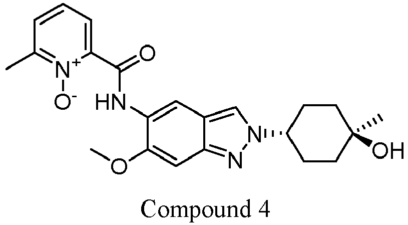

Anhydrous tetrahydrofuran (12 L) and *cis*-4-(6-methoxy-5-amino-indazol-2-yl)-1-methyl-cyclohexanol (1100.00 g, 4 mol) were sequentially added to a bilayer glass reactor (20 L). The internal temperature of the reactor was maintained at 20-25 °C. 2-(7-Azabenzotriazole)-*N*,*N*,*N*',*N*'-tetramethyluronium hexafluorophosphate (1551.38 g, 4.08 mol) and *N*,*N*-diisopropylethylamine (672.10 g, 5.2 mol) were sequentially added. After the addition, the reaction system was stirred for 3 h. The reaction was completed as indicated by HPLC (high performance liquid chromatography). Water (5 L) was added to the reaction system. The mixture was then warmed to 30-35 °C, concentrated *in vacuo* until a certain volume (7 L) was reached, cooled to 0-10 °C, stirred for another 5 h, and filtered. The filter cake was recrystallized in ethanol, and the crystal was collected by filtration and dried to give compound 4 (1503.9 g, 91.7% yield, 99.6% purity as indicated by HPLC (214 nm)). ¹H NMR (400 MHz, DMSO-*d₆*): δ 14.16 (s, 1H), 8.78 (s, 1H), 8.34 (s, 1H), 8.32-8.30 (m, 1H), 7.77 (d, J = 7.6 Hz, 1H), 7.58 (t, J = 8.0 Hz, 1H), 7.13 (s, 1H), 4.45 (s, 1H), 4.43-4.40 (m, 1H), 3.95 (s, 3H), 2.53 (s, 3H), 2.09-2.00 (m, 4H), 1.68-1.58 (m, 4H), 1.22 (s, 3H); LCMS m/z = 411.1 [M+H]⁺.
Biological test example: Inhibitory effects of example compounds 1, 2, 3 and 4 of the present disclosure against human IRAK4 kinase activity
Major materials
   ATP (Sigma), DMSO (Sigma), EDTA (Sigma), HEPES (Sigma), DTT (Sigma), and Brij-35 (Sigma)
Procedures
   The inhibitory activity of the compounds against IRAK4 at the Km concentration of ATP was measured in IRAK4 MSA (Mobility-Shift Assay, a mobility detection of microfluidic chip technology).

A recombinant fusion protein of N-terminal GST (glutathione-S-transferase) and human IRAK4 was used as enzyme (GST-IRAK4, kinase IRAK4 (Carna)) at a final concentration of 1 nM; ATP (Sigma) was at a final concentration of 37 µM; the substrates used for the kinase reaction were 5-FAM (5-carboxyfluorescein)-labeled polypeptide (5-FAM-IPTSPITTTYFFFKKK-COOH) and substrate peptide FAM-P8 (GL Biochem) at final concentrations of 5 µM.

In this assay, a 500 µM stock solution of the compounds was prepared in 100% DMSO, and serially 4-fold diluted to the 10^{th} concentrations with 100% DMSO, followed by a 10-fold dilution with the compound buffer (50 mM HEPES, pH 7.5, 0.00015% Brij-35) to give intermediate dilutions of the compounds at final concentrations of 10 µM-0.04 nM containing 10% DMSO. The intermediate dilutions was transferred into a black 384-well plate at a volume of 5 µL.

Kinase IRAK4 was diluted to 2.5 nM with the kinase buffer (50 mM HEPES, pH 7.5, 0.00015% Brij-35, 2 mM DTT). 10 µL of the IRAK4 dilution was transferred to the 384-well plate and co-incubated with the compound for 10-15 min.

The substrate and ATP were diluted to 12.5 µM and 92.5 µM with reaction buffer (50 mM HEPES, pH 7.5, 0.00015% Brij-35, 10 mM MgCl₂), respectively. 10 µL of the dilutions was transferred to the 384-well plate and incubated at 28 °C for 1 h. The reaction was terminated by adding 25 µL of 50 mM EDTA to the 384-well plate.

The inhibition rates of IRAK4 by the compounds were calculated by measuring the conversion rate of phosphorylation of the substrate using a Caliper EZ Reader (PerkinElmer) and the IC₅₀ was calculated by XL-fit software.

The IC₅₀ values of the example compounds 1, 2, 3 and 4 in inhibiting human IRAK4 kinase activity are shown in Table 1.

**Table 1. IC₅₀ of the example compounds against human IRAK4 kinase activity**

| **Compound ID** | **IC₅₀ (nM)** |
|---|---|
| Compound 1 | 1.1 |
| Compound 2 | 2.5 |
| Compound 3 | 3.3 |
| Compound 4 | 4.6 |

As can be seen from Table 1, the compounds prepared in the examples of the present disclosure have superior inhibitory effects on human IRAK4 activity.

Examples of the present disclosure have been described above. However, the present disclosure is not limited thereto. Any modification, equivalent, improvement and the like made without departing from the spirit and principle of the present disclosure shall fall within the protection scope of the present disclosure.

## Claims

1. A method for preparing a compound of formula (I), a salt or another derivative thereof, comprising the following steps: 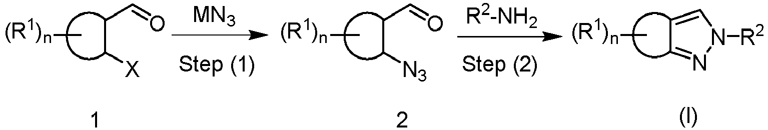
wherein, 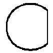 represents a ring system, such as a ring system selected from: C₃₋₄₀ cycloalkyl, 3- to 20-membered heterocyclyl, C₆₋₂₀ aryl and 5- to 20-membered heteroaryl;
each R¹ is the same or different and is independently selected from an organic group;
R² is selected from an organic group;
n is selected from an integer, such as 0, 1, 2, 3, 4, 5, 6, 7 and 8;
X is selected from a halogen, such as F, Cl, Br and I;
MN₃ is selected from an azide reagent, such as sodium azide, trimethylsilyl azide and diphenylphosphoryl azide;
preferably, the step (1) and/or step (2) are carried out in the presence of an alcohol-type solvent;
preferably, the step (2) may be carried out in the presence of an acid;
preferably, the step (2) may be carried out at a heating condition;
preferably, the alcohol-type solvent may be selected from one, or a mixture of two or more of methanol, ethanol, *n*-propanol, isopropanol, *n*-butanol, isobutanol and *tert*-butanol, preferably from one, or a mixture of two or more of methanol, ethanol and isopropanol; preferably, the alcohol-type solvent is an anhydrous solvent;
preferably, the acid may be an organic acid or inorganic acid; for example, the acid is selected from one, or a mixture of two or more of formic acid, acetic acid, propionic acid, sulfuric acid and *p*-toluenesulfonic acid.

2. The method according to claim 1, wherein each R¹ is the same or different and is independently selected from a halogen, CN, OH, NO₂, and the following groups unsubstituted or optionally substituted with one, two or more R^{a}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, -O(CH₂)ₘR³, -S(CH₂)ₘR³, -NR⁴R⁵, -C(O)R⁶, -S(O)₂R⁶, -OC(O)R⁷, -OS(O)₂R⁷ and -S(O)(NR⁸)R⁹; wherein m is selected from an integer, such as 0, 1, 2, 3, 4, 5, 6, 7 and 8;
each R³ is the same or different and is independently selected from C₁₋₄₀ alkyl, C₁₋₄₀ alkoxy, C₁₋₄₀ alkylthio, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
R⁴ and R⁵ are the same or different and are each independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5-to 20-membered heteroaryl, 3- to 20-membered heterocyclyl, -C(O)R⁶ and -S(O)₂R⁶;
or R⁴ and R⁵, together with an N atom connected thereto, form 5- to 20-membered heteroaryl or 3- to 20-membered heterocyclyl;
each R⁶ is the same or different and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, -OR³, -SR³ and NR⁴R⁵;
each R⁷ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
each R⁸ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
each R⁹ is the same or different, and is independently selected from H, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
R² is selected from the following groups unsubstituted or optionally substituted with one, two or more R^{b}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
each R^{a}, R^{b} is the same or different and is independently selected from a halogen, CN, OH, SH, oxo (=O or forming a nitrogen oxide), NO₂, and the following groups unsubstituted or optionally substituted with one, two or more R^{c}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, -OR³, -SR³, -NR⁴R⁵, -C(O)R⁶, -S(O)₂R⁶, -OC(O)R⁷, -OS(O)₂R⁷, -SOR⁷ and -S(O)(NR⁸)R⁹;
each R^{c} is the same or different and is independently selected from a halogen, CN, OH, SH, oxo (=O or forming a nitrogen oxide), NO₂, C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl and C₁₋₄₀ haloalkyl.

3. The method according to claim 1 or 2, wherein R¹ is selected from NO₂, NH₂, and -OR³ unsubstituted or optionally substituted with one, two or more R^{a};
preferably, R³ may be selected from C₁₋₄₀ alkyl, such as C₁₋₆ alkyl;
preferably, R² may be selected from the following groups, which are unsubstituted or optionally substituted with one, two or more R^{b}: -C₃₋₄₀ cycloalkyl-OH and -C₃₋₄₀ cycloalkyl-C₁₋₄₀ alkyl-OH, such as -C₄₋₆ cycloalkyl-OH and -C₄₋₆ cycloalkyl-C₁₋₆ alkyl-OH, such as 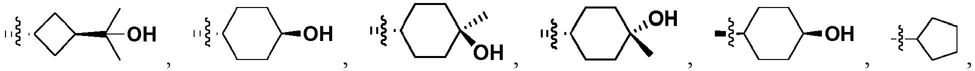 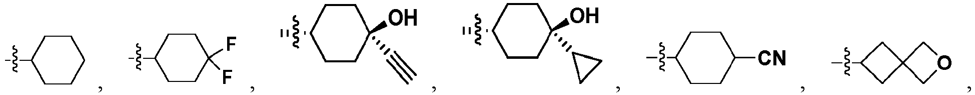 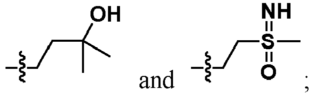
preferably, n is an integer selected from 0 to 4, preferably 2;
preferably, where n is 2, two same or different R¹ may substitute at ortho or non-ortho positions.

4. The method according to any one of claims 1-3, wherein after the reaction of the step (1) is completed, no post-treatment is required and the reaction of the step 2) can be directly carried out;
preferably, in the step (2), a molar ratio of the compound 2 to R-NH₂ is 1:(0.5-5), such as 1:(0.8-3);
preferably, in the step (2), a molar ratio of the compound 2 to the acid is 1:(0.5-8), such as 1:(0.8-5);
preferably, the reaction time of the step (2) may be 1-24 h, such as 2-18 h;
preferably, the reaction of the step (2) may be carried out at a temperature of 40-120 °C, such as 60-100 °C, for example, 70-85 °C;
preferably, after the reaction of the step (1) is completed, the reaction of the step (2) is directly carried out without isolating the compound of formula 2;
preferably, after the reaction of the step (2) is completed, the compound of formula (I) is isolated without using column chromatography;
preferably, after the reaction of the step (2) is completed, the compound of formula (I) can be isolated by using methods such as filtering the reaction system through celite and then concentrating the resulting filtrate, adding water to the reaction system and then filtering the resulting mixture, and adding water to the reaction system and then concentrating the resulting mixture.

5. The method according to any one of claims 1-4, comprising the following steps: 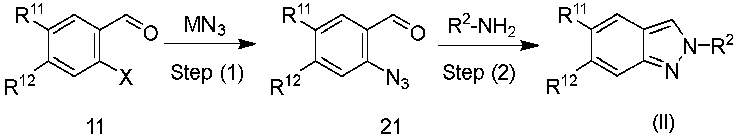
wherein, R¹¹ and R¹² are the same or different and are each independently selected from the definitions of R¹ described in any one of claims 1-3;
R², X and M are each independently as defined in any one of claims 1-3;
preferably, R¹¹ is selected from NO₂;
preferably, R¹² is selected from methoxy;
preferably, R² is selected from 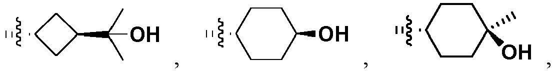 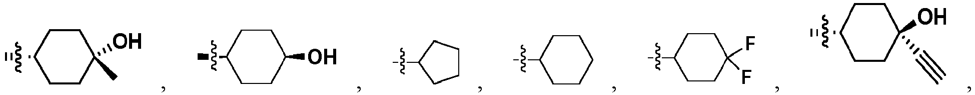 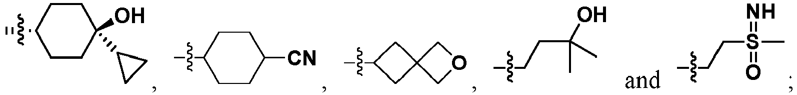
preferably, X is selected from F;
preferably, MN₃ is selected from NaN₃;
preferably, the compound of formula (I) is preferably selected from the compound of formula (II);
preferably, the method is for preparing an indazole-type compound, such as the compound of formula (II), a salt or another derivative thereof.

6. Use of the method according to any one of claims 1-5 in preparing an indazole derivative, such as an indazole derivative having inhibitory activity against IRAK.

7. A method for preparing the derivative or salt of the compound of formula (I) according to claim 1, comprising derivatizing a group in the compound of formula (I) or the salt thereof with a reagent after the above method, wherein:
preferably, a method for preparing a compound of formula 4 comprising:
(a1) subjecting a compound of formula (I-3) to a reduction reaction to give compound 3; and
(a2) subjecting compound 3 and Rₛ-COOH to a condensation reaction to give compound 4; 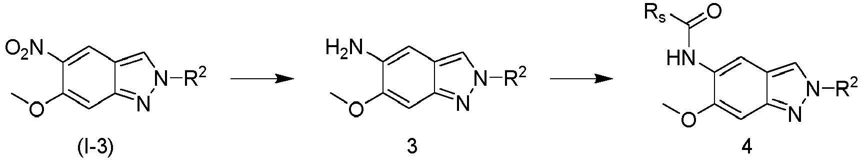 wherein, Rₛ is selected from the following groups unsubstituted or optionally substituted with one, two or more R^{a}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl and 3- to 20-membered heterocyclyl;
R² and R^{a} are independently as defined in any one of claims 1-3;
preferably, Rₛ is selected from 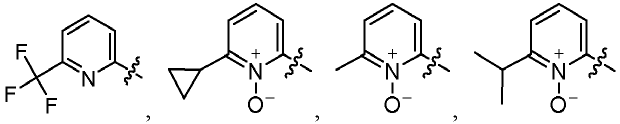 and

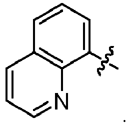

8. A composition comprising a compound of formula 1, a compound of formula 2 and an alcohol-type solvent;
or
comprising a compound of formula 2, a compound of formula (I), an alcohol-type solvent and an acid;
or
comprising a compound of formula 1, a compound of formula 2, a compound of formula (I), an alcohol-type solvent and an acid;
wherein preferably, the composition further optionally comprises or does not comprise at least one selected from the following components: MN₃ and R²-NH₂; wherein M and R² are independently as defined in any one of claims 1-3.

9. The method according to any one of claims 1-5, wherein the compound of formula (I) is a racemate, a stereoisomer, a conformer or a tautomer.

10. The method according to any one of claims 1-5, wherein an element in the structures of the compound of formula 1, the compound of formula 2 and the compound of formula (I) may be optionally replaced by isotopes thereof; for example, ¹H may be replaced by ²H.
